# EUROPEAN PATENT APPLICATION

(11) **EP 4 079 844 A1**
(43) Date of publication of application: **26.10.2022**
(21) Application number: 20902053.6
(22) Date of filing: 17.12.2020
(51) Int. Cl.: C12N 5/079, G01N 33/50

(54) **METHOD OF MICROGLIA DIFFERENTIATION CAPABLE OF SECURING LARGE QUANTITY OF MICROGLIA BY USING 3D ORGANOIDS FROM HUMAN PLURIPOTENT STEM CELLS**

(30) Priority: 17.12.2019 KR 20190168516
(71) Applicant: Corestem Co., Ltd., Seongnam-si, Gyeonggi-do 13486 (KR)
(72) Inventor: LEE, Sang Hun, Seoul 04763 (KR); KIM, Seung Won, Seoul 04763 (KR); CHANG, Mi Yoon, Seoul 04763 (KR); KIM, Su Hyun, Seoul 04763 (KR)
(74) Representative: HGF
(86) International application number: PCT/KR2020/018569
(87) International publication number: WO 2021/125839

(57) **Abstract**

The present invention relates to a differentiation method for obtaining a large quantity of microglia by patterning, proliferating, culturing, and inducing the differentiation of yolk sac-mimic 3D organoids prepared from human pluripotent stem cells, wherein the microglia thus obtained in a large quantity exhibit significantly superior effects in terms of yield, purity, and storage stability compared to cells differentiated by existing differentiation methods, and thus may be utilized in research on lesions and therapeutic mechanisms of brain diseases, and drug screening platforms.

## Description

### Technical Field

The present invention relates to a method for differentiation of microglia to secure a large quantity of microglia by using yolk sac-mimic organoids prepared from human pluripotent stem cells.

### Background Art

Most of the modern research on human brain diseases has been conducted using rodent neurons. The reason is that it is difficult to obtain human brain neurons and there is a certain degree of similarity between the rodent genes and the human genes. However, recently, as research in the field of stem cells has progressed, methods for differentiating human brain nervous system cells from embryonic stem cells have been developed, and brain disease research has gradually shifted from studies using rodent animal cells to studies using human cells directly. In addition, as research in the genomic field has recently been developed, it has been revealed that the similarity between human and laboratory animal genes is considerably lower than expected, especially in microglia. This suggested a problem in that the interaction between the human brain nervous system cells and the laboratory animal brain nervous system cells made it difficult to significantly represent lesions and therapeutic effects that actually occur in the human brain due to genetic differences between species.

In particular, microglia play a pivotal role in inflammation within brain tissue, among other things. Therefore, understanding this is very important for the identification of the pathogenesis of human diseases and the development of drugs for the diseases. Nevertheless, compared to other cells of the brain and nervous system, the differentiation method of microglia is different from other cells of the brain and nervous system, and a stably established differentiation method has not been studied up to now, so the development of such a differentiation method is urgently needed.

The brain nervous system is largely composed of neurons, astrocytes, oligodendrocytes, and microglia. Neurons, astrocytes, and oligodendrocytes are all generated from neural stem cells derived from ectoderm during development, but microglia are derived from the yolk sac through a process of primitive hematopoiesis during development. This is a process distinct from definitive hematopoiesis, in which hematopoietic stem cells that produce blood cells are produced. Nevertheless, most of the protocols developed up to now differentiate through a process of definitive hematopoiesis, which is the same developmental process as hematopoietic stem cells.

A method for differentiation from human pluripotent stem cells into microglia has been studies, developed, and reported [Non-Patent Documents 1 and 2], but the following problems are presented.
(1) It takes a considerable amount of time to obtain microglia.
   It takes 75 days to obtain mature microglia in the protocol of Non-Patent Document 1, and it takes 38 days to obtain mature microglia in the protocol of Non-Patent Document 2. If it takes more than 38 to 75 days to obtain cells, it is important to advance the differentiation period for securing cells smoothly, since a preparation period of at least about 40 days is required for each experiment.
(2) Oxygen concentration control instrument and many kinds of protein treatments are required.
   In the case of the protocol of Non-Patent Document 2, a hypoxia condition is applied for the first 4 days in order to increase the yield. The number of proteins processed during the differentiation period is inefficiently as high as 14.
(3) Yield is low, or a cell sorting process is separately required.
   In the case of the protocol of Non-Patent Document 1, only 0.5-4×10⁶ microglia are obtained on day 74 starting with 1×10⁶ iPS cells. In the case of the protocol of Non-Patent Document 2, the amount of microglia that can be obtained starting with 1×10⁶ iPS cells is as high as 30×10⁶ to 40×10⁶, but the cell sorting process using FACS should be performed during the protocol. Through this cell sorting process, cell damage and cell loss are inevitably accompanied.

### Prior Art Document

### Non-Patent Document

Non-Patent Document 1: Julien Muffat, Rudolf Jaenisch. et al. 2016, Efficient derivation of microglia-like cells from human pluripotent stem cells. Nature Medicine. 22, pages 1358-1367
Non-Patent Document 2: Edsel M.Abud, MathewBlurton-Jones. et al. 2017, iPSC-Derived Human Microglia-like Cells to Study Neurological Diseases. Neuron. Volume 94, Issue 2, 19 April 2017, Pages 278-293.e9

### Detailed Description of Invention

### Technical Problem

Accordingly, the present inventors have developed a protocol that most closely resembles the actual *in vivo* development process because it induces the differentiation into primitive streak HSCs by blocking the Wnt pathway by adding XAV939 to the medium when culturing yolk sac-mimic organoids, which are prepared from human pluripotent stem cell. In particular, the present inventors have developed a differentiation method that can acquire a large quantity of pure microglia without any special cell sorting process so that the microglia generated by replacing the medium with brain cell culture conditions during culture are ejected into the culture solution. Based on the above, the present inventors completed the present invention.

### Solution to Problem

Therefore, an object of the present invention is to provide a medium composition for inducing differentiation of microglia, the medium composition further comprising a compound represented by Formula 1 below.

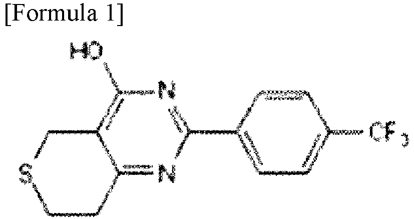

In addition, another object of the present invention is to provide a method for differentiation of microglia to obtain a large quantity of microglia, the method comprising:
preparing yolk sac-mimic organoids from human pluripotent stem cells; and
replacing an NGD (neuroglia differentiation) medium with an N2 supplement medium while culturing the organoids in the NGD (neuroglia differentiation) medium;
wherein a compound represented by Formula 1 below is further included in the NGD medium when culturing in the NGD medium.

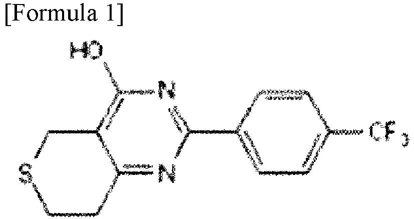

In addition, another object of the present invention is to provide a composition for preventing or treating a neurodegenerative disease or an inflammatory degenerative disease, the composition comprising an anti-inflammatory cytokine derived from the differentiated microglia obtained by the method.

In addition, another object of the present invention is to provide a drug screening method using the differentiated microglia obtained by the method.

### Effects of Invention

The method for differentiation of microglia according to the present invention may be said to be the protocol that most resembles the actual *in vivo* development process because differentiated microglia block the Wnt pathway through XAV939 to induce the differentiation into primitive streak HSCs. In addition, in exisiting methods for differentiation of microglia, 5% hypoxia is initially applied to increase the cell yield, but in the present invention, the yield of microglia obtained without applying the hypoxia method is higher than that of other control groups.

It is a system that generates organoids that mimic the yolk sac in the developmental stage and replaces the medium with brain cell culture conditions from day 9 to day 11 of culture (differentiation) so that the microglia efficiently generated are ejected into the culture solution. Pure microglia may be acquired without any special cell sorting process. That is, more than 90% purity of microglia could also be obtained without cell sorting such as FACS or MACS. Moreover, the microglia may also be cryopreserved. The method for differentiation of microglia according to the present invention may be utilized as a platform for research on lesions and therapeutic mechanisms of brain diseases, and drug screening through a single cell system, or by developing a microscopic three-dimensional brain structure system similar to that of an adult human brain through co-culture with other brain neurons, astrocytes, and oligodendrocytes.

### Brief Description of Drawings

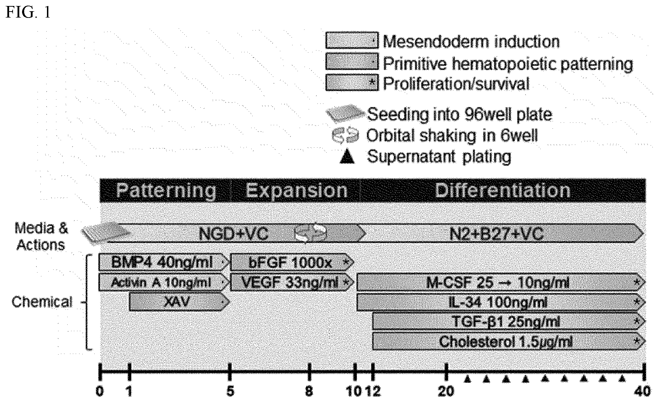

FIG. 1 illustrates a detailed protocol for differentiating microglia using the yolk sac organoids according to the present invention [x-axis indicates days of culture (differentiation)].
FIG. 2 is a photograph of the organoids from day 1 to day 9 after differentiation (culture) derived from iPSCs that are developing in the yolk sac.
FIG. 3 is a photograph confirming the cross-section by immunocytochemistry analysis by cutting the organoids into sections after freezing in order to confirm whether primitive hematopoietic stem cells (primitive HSCs) are actually generated inside the yolk sac organoids.
FIG. 4 is a photograph confirming the cross-section of the organoids by immunocytochemistry analysis after adherent culture by degrading the organoids to a single cell level.
FIG. 5 illustrates a result obtained by confirming the amount of gene markers expressed in the early stage of microglia differentiation by collecting RNA of the yolk sac organoids on day 14 of differentiation (culture) by rt-PCR.
FIG. 6 illustrates the change in the shape of microglia ejected from the yolk sac organoids. On day 10 after the start of differentiation (culture), the medium is replaced with the N2 medium containing M-CSF and IL-34, which mimics the environment in which microglia escape from the yolk sac and penetrate into the brain.
FIG. 7 is a photograph taken after collecting the microglia ejected from the yolk sac organoids that were differentiated (cultured) for 24 days and attaching to the cell culture dish.
FIG. 8 illustrates a result obtained by confirming the expressions of the mature microglia marker genes (Iba1; microglia, CD11b; macrophage and microglia, CX3CR1; microglia-enriched protein) in each of the microglia ejected from the yolk sac organoids on day 32 of differentiation (culture) (supernatant, sup) and the yolk sac organoids by qPCR.
FIG. 9 illustrates a result obtained by analyzing the cross-section of the yolk sac organoids that were differentiated (cultured) for 38 days by immunocytochemistry analysis.
FIG. 10 illustrates a result obtained by confirming the generation of microglia progenitor cells by a microscope [①: a photograph of cells ejected from the organoids plated on a cell culture dish on day 24 of differentiation (culture); ②: a photograph when further cultured for +11 days in the same well as in photograph ①; ③: a photograph when further cultured for +29 days in the same well as in photograph ① (cells attached to the dish and floating cells); and ④: a photograph of the cells by collecting the floating cells again in photograph ③ and plating on a cell culture dish].
FIG. 11 illustrates the expression of the microglia marker in microglia progenitor cells. The microglia candidate cell population having an excellent proliferative capacity does not proliferate immediately after escaping from the organoids, but proliferates after a certain period of time. IBA1 staining confirmed the possibility that proliferating cells were microglia progenitor cells (white arrow indicates proliferating cells).
FIG. 12 illustrates the expression of the microglia marker in microglia progenitor cells. It illustrates a result obtained by confirming by immunocytochemistry analysis that both the microglia progenitor cell candidate having an excellent proliferative capacity and microglia express CX3CR1, an important marker of microglia.
FIG. 13 illustrates a result obtained by confirming the response to toxic stimuli by inflammatory response markers when astrocytes are cultured alone or when astrocytes and microglia are co-cultured.
FIG. 14 illustrates a result obtained by confirming the phagocytic activity of microglia differentiated by the differentiation method according to the present invention using fluorescent beads and immunocytochemistry analysis.
FIG. 15 illustrates a result obtained by confirming the phagocytic activity of microglia differentiated by the differentiation method according to the present invention by phagocytic activity analysis experiment using pHrodo and immunocytochemistry analysis. FIG. 15A illustrates a result obtained by treating with pHrodo, and FIG. 15B illustrates a result obtained by quantifying the amount of pHrodo accumulated in the cells through intensity analysis.
FIG. 16 illustrates the cryopreservation possibility of microglia differentiated by the differentiation method according to the present invention.
FIG. 17 is a graph showing the number of harvests of microglia obtained by ejection on days 21 to 39 of differentiation (culture) according to the differentiation method according to the present invention. It is a result obtained by collecting cells (microglia + microglia progenitor cells) released from the yolk sac-mimic organoids once every 2 days and attaching to one well (9.6 cm²) of a 6 well plate from day 21 to day 39, and then harvesting the microglia floating in each well again once every 2 days and measuring them.
FIG. 18 illustrates a result obtained by comparative analysis of the gene expression data (public data) of human fetal microglia through Non-Patent Document 1 and the gene expression data of microglia (red box) and H9 embryonic stem cells obtained by the differentiation method of the present invention.

### Best Mode for Carrying out the Invention

Hereinafter, the present invention will be described in more detail.

The present invention relates to a method for differentiation of microglia to obtain a large quantity of microglia by using yolk sac-mimic organoids prepared from human pluripotent stem cells.

As used herein, the term "pluripotent stem cell (PSC)" refers to a stem cell capable of inducing differentiation into any type of cell constituting the body, and the pluripotent stem cell includes an embryonic stem cell (ESC) and an induced pluripotent stem cell (Ipsc, dedifferentiated stem cell).

As used herein, the term "yolk sac" refers to a pocket-shaped structure composed of a (ploid) ectodermal layer containing egg yolk in the embryo of a telolecithal egg or polylecithal egg animal, that is, a thin pocket enclosing the embryo. It may be observed in fertilized ovums. It plays a role in supplying blood to the embryo in the egg yolk. In humans, as the embryo grows, most of the yolk sac is fused to the early intestine.

As used herein, the term "organoid" refers to a 'mini-organ like' made to have a minimum function using stem cells, and is characterized in that it is made in a three-dimensional structure and may create an environment similar to an actual body organ even in a laboratory. That is, "organoid" refers to cells having a 3D stereostructure, and refers to a model similar to organs such as nerves and intestines prepared through an artificial culture process that is not collected or acquired from animals and the like. The origin of the cells constituting it is not limited. The organoid may have an environment that is allowed to interact with the surrounding environment in the process of cell growth.

An organoid may generally be prepared by culturing human pluripotent stem cells. Specifically, it may induce differentiation from induced pluripotent stem cells derived from Parkinson's disease into neuroectodermal spheres, or the differentiation into intestinal organoids through step-by-step differentiation from induced pluripotent stem cells derived from Parkinson's disease into definitive endoderm and hindgut.

As used herein, the term "microglia" is also referred to as a glial cell or neuroglia, and is a neuroglia responsible for immune function in the brain. In general, it is mesodermal and is considered to be derived from monocytes. Microglia appear around the ventricle and meninges in the late stage of fetal development, gradually move into the cerebral parenchyma, and reach the peak at 2 weeks of age, and then decrease in number to become branched resting cells. In the mature brain, it accounts for about several percent of all neuroglia. Amoeba-shaped and rod-shaped microglia increase during inflammation or neurodegeneration. Morphologically, they are similar to macrophages, and most of their markers are used for the identification of microglia. They have the migration ability and phagocytic activity, and in addition to the function of processing waste during the development phase, they also have a function as an inflammatory cell by producing reactive oxygen species, nitrogen monoxide, acid phosphatase, and prostaglandins. As there are only class II major histocompatibility antigens or CD4 antigens in the brain, they play an important role in neuro-immune interactions as immunoregulatory cells in that they actively produce cytokines. It is the smallest among gliacytes and is a special type of macrophage that protects neurons through phagocytosis by migrating microglia to damaged nerves. Since the origin of microglia is an endoderm-derived hematopoietic stem cell, there is little connection with other cells of the nervous system.

As used herein, the term "patterning" refers to the preparation of an organoid such that, when preparing an organoid, the cell population with fate having the property of the origin tissue of the cell to be finally extracted from among the brain detailed tissues is contained as a plurality of cell populations (target cell enriched). In addition, patterning markers include CD34, CD41, CD235a, PU.1, and the like.

As used herein, the term "differentiation" refers to a phenomenon in which the structure or function of cells is specialized to each other during division, proliferation, and growth, that is, a change in the form or function of cells, tissues, and the like of organisms in order to perform a given task. In general, it is a phenomenon in which a relatively simple system is divided into two or more qualitatively different subsystems. For example, in ontogenesis, a qualitative difference between parts of a biological system that was initially almost homogeneous, such as a distinction of a head or a trunk between egg parts that were initially homogeneous, or a distinction of a muscle cell or a nerve cell in cells, or as a result, a state of being divided into qualitatively distinguishable subdivisions or subsystems is called differentiation.

By preparing yolk sac-mimic organoids from human pluripotent stem cells, that is, obtaining organoids containing the maximum amount of each target cells, patterning and proliferating the organoid tissue, and replacing the liquid medium with brain cell culture conditions during culture, it was confirmed that just as microglia penetrate into the brain at the actual development stage, after a certain culture period, in the CSF (cerebrospinal fluid) environment, only microglia efficiently escaped from the organoids into the culture solution by taking a strategy to change the culture conditions in the brain, and a large quantity of microglia having a high purity may be obtained. Since there is the proliferating cell population in the cell population that has escaped in this way, it is possible to continue to separate the microglia in about 30 to 40 days.

Therefore, in the present invention, a medium composition and differentiation method capable of obtaining a large quantity of microglia having a high purity were established by replacing the medium with brain cell culture conditions during culturing of the prepared yolk sac organoids.

According to one embodiment of the present invention, a medium composition for inducing differentiation of microglia includes a medium composition for inducing differentiation of microglia that further comprise a compound represented by Formula 1 below.

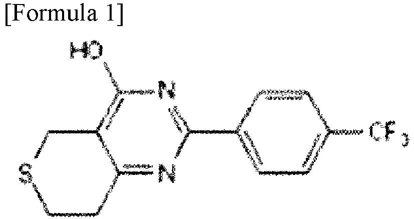

The compound represented by Formula 1 is XAV939 (CAS 284028-89-3), which is referred to as 3,5,7,8-tetrahydro-2-[4-(trifluoromethyl)phenyl]-4H-thiopyrano[4,3-d]pyrimidin-4-one. XAV939 is a potent inhibitor of beta-catenin, TNKS1 and TNKS2.

XAV939 induces the differentiation into primitive streak HSCs by blocking the Wnt pathway.

The medium for inducing differentiation of microglia is preferably an NGD (neuroglia differentiation) medium.

The NGD (neuroglia differentiation) medium refers to a medium composition comprising Gem21, Neuroplex N2, Albumax I, sodium chloride, sodium pyruvate, biotin, lactic syrup, GlutaMAX, ascorbic acid, and/or penicilin/streptomycin. Reference may be made to the literature [Julien Muffat, Rudolf Jaenisch. et al. 2016, Efficient derivation of microglia-mimic cells from a human pluripotent stem cell. Nature Medicine. 22, pages 1358-1367].

BMP4 (bone morphogenetic protein 4) and activin A may be further included in the medium for inducing differentiation of microglia.

In addition, bFGF (basic fibroblast growth factor) and VEGF (vascular endothelial growth factor) may be further included in the medium for inducing differentiation of microglia.

The medium composition may be used for obtaining a large quantity of microglia having a high purity without the need for hypoxia equipment and many protein components.

According to one embodiment of the present invention, it includes a method for differentiation of microglia to obtain a large quantity of microglia, the method comprising:
preparing yolk sac-mimic organoids from human pluripotent stem cells; and
replacing an NGD medium with an N2 supplement medium while culturing the organoids in the NGD medium;
wherein a compound represented by Formula 1 below is further included in the NGD medium when culturing in the NGD medium.

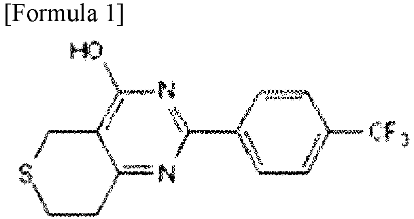

The time to replace the NGD medium with an N2 supplement medium is preferably from day 9 to day 11 of culture.

BMP4 (bone morphogenetic protein 4) and activin A may be further included in the NGD medium.

In addition, bFGF (basic fibroblast growth factor) and VEGF (vascular endothelial growth factor) may be further included in the NGD medium.

M-CSF (macrophage colony stimulating factor) and IL-34 (interleukin 34) may be further included in the N2 supplement medium.

Cholesterol and TGF-β1 (transforming growth factor beta 1) may be further included in the N2 supplement medium.

A medium composition comprising 7 types of proteins, XAV-939 and cholesterol without an oxygen concentration control instrument may be used for obtaining a large quantity of microglia having a high purity without the need for an oxygen concentration control instrument and many protein components.

According to one embodiment of the present invention, performing cryopreservation may be further included.

The cryopreservation refers to storage at -250°C to -150°C for 3 to 6 months.

When the microglia implemented in the present invention were cryopreserved and thawed again, it was confirmed that more than 70% of the cells were alive. Therefore, the microglia obtained by the method for differentiation of microglia according to the present invention could also be cryopreserved.

In the differentiation method according to the present invention, it is preferable to culture in an incubator of 1-10% CO₂. It does not require hypoxic culture as in the conventional differentiation method, and may be performed in the same manner as in general cell culture conditions. In addition, it is possible to obtain microglia with high yield and purity without performing a cell sorting process as in the conventional differentiation method.

Specifically, mature microglia may be obtained in about 30 to 40 days. In particular, it is possible to produce cells 45 to 55 days earlier than in Non-Patent Document 1 and about 8 to 18 days earlier than in Non-Patent Document 2.

In addition, microglia were successfully cultured using only 7 types of proteins, 1 type of chemical (XAV-939), and 1 type of an organic compound (cholesterol) without an oxygen concentration control instrument. Therefore, this method is a more economical and efficient differentiation method compared to the conventional method.

The protocol of Non-Patent Document 2, which currently has the highest yield, produces only 30 to 40 times the number of microglia on day 38 compared to the number of cells added at the start, whereas the protocol of the differentiation method according to the present invention may produce 5.5 to 6.5 times the number of mature microglia on day 30 compared to the initial number of cells (starting with 2×10⁶ iPS cells), and about 50 times if accumulated by day 40. Moreover, unlike the protocol of Non-Patent Document 2, the protocol of the differentiation method according to the present invention shows a purity of up to 90% without FACS cell sorting, so it is possible to obtain a stable amount of microglia without damaging the cells.

In microglia, activation of microglia may be divided into two types, M1 and M2, and the M1 response releases factors such as inflammatory cytokines. In other words, it exacerbates brain damage by generating neurotoxic substances. These neurotoxic substances include inflammatory cytokines such as IL-1β, TNF-α, IL-6, glutamate, and NO. In the M2 response, they protect adjacent cells by removing cellular debris and releasing nutrient factors for brain repair.

As such, the M2 response recovers tissues damaged by the inflammatory response and forms or repairs blood vessels for nutrient supply. Substances that play this role are anti-inflammatory cytokines such as IL-4, IL-10, TNF-β, IGF-1, and IL-33.

Therefore, anti-inflammatory cytokines derived from microglia may be utilized to prevent or treat a neurodegenerative disease or an inflammatory degenerative disease.

One embodiment of the present invention includes a composition for preventing or treating a neurodegenerative disease or an inflammatory degenerative disease, comprising an anti-inflammatory cytokine or a growth factor derived from microglia obtained by the differentiation method.

The anti-inflammatory cytokine may be at least one selected from the group consisting of IL-4, IL-10, IL-33, TNF-β and IGF-1.

The composition of the present invention may further comprise appropriate carriers, excipients and diluents commonly used in the preparation of pharmaceutical compositions.

The composition of the present invention may be administered orally or parenterally. For parenteral administration, it is preferable to select an injection method for external application or intraperitoneal injection, intrarectal injection, subcutaneous injection, intravenous injection, intramuscular injection or intrathoracic injection, but is not limited thereto.

The composition of the present invention may be formulated in the form of oral formulations such as powders, granules, tablets, capsules, suspensions, emulsions, syrups, and aerosols, external formulations, suppositories and sterile injection solutions according to conventional methods, respectively. Carriers, excipients and diluents that may be included in the composition include lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate and mineral oil. In case of formulation, it is prepared using commonly used diluents or excipients such as fillers, extenders, binders, wetting agents, disintegrants, and surfactants. Solid preparations for oral administration include tablets, pills, powders, granules, capsules, and the like, and these solid preparations are prepared by mixing at least one excipient, for example, starch, calcium carbonate, sucrose or lactose, gelatin, and the like with the mixed herbal medicine. In addition to simple excipients, lubricants such as magnesium stearate and talc are also used. Liquid preparations for oral administration include suspensions, internal liquids, emulsions, syrups, and the like. In addition to water and liquid paraffin, which are commonly used simple diluents, various excipients, for example, wetting agents, sweeteners, perfuming agents, preservatives, and the like may be included.

Preparations for parenteral administration include sterilized aqueous solutions, non-aqueous solutions, suspensions, emulsions, freeze-dried preparations, and suppositories. As a non-aqueous solvent and a suspending agent, propylene glycol, polyethylene glycol, vegetable oils such as olive oil, and injectable esters such as ethyl oleate may be used. As the base of the suppositories, witepsol, macrogol, tween 61, cacao butter, laurin butter, glycerogelatin, and the like may be used.

A preferred dosage of the composition of the present invention may vary depending on the condition and body weight of the patient, the severity of the disease, the form of the drug, the route of administration, and the duration, but may be appropriately selected by one of ordinary skill in the art. However, for a desirable effect, the composition is preferably administered at 0.0001 to 1 g/kg per day, more preferably at 0.001 to 200 mg/kg per day, but is not limited thereto. The administration may be performed once a day or divided into several times a day. The dosage does not limit the scope of the present invention in any aspect.

As used herein, the term "treatment" refers to any action that inhibits, alleviates, or advantageously alters a clinical situation related to a disease. In addition, treatment may mean increased survival compared to the expected survival rate if not receiving treatment. Treatment simultaneously includes prophylactic means in addition to therapeutic means.

The neurodegenerative disease may include, for example, any one selected from the group consisting of Parkinson's disease, dementia, Alzheimer's disease, Huntington's disease, amyotrophic lateral sclerosis, memory loss, myasthenia gravis, progressive supranuclear palsy, multiple system atrophy, essential tremor, corticobasal degeneration, diffuse Lewy body disease and Pick disease, but is not limited thereto.

The inflammatory degenerative disease may include any one selected from the group consisting of dementia, Lewy body dementia, frontotemporal dementia, white matter degeneration, adrenoleukodystrophy, multiple sclerosis and Lou Gehrig's disease, but is not limited thereto.

In another embodiment, the present invention provides a method for treating a neurodegenerative disease or an inflammatory degenerative disease, comprising administering to a subject a therapeutically effective amount of an anti-inflammatory cytokine derived from microglia.

As used herein, the term "subject" refers to a vertebrate to be treated, observed or experimented, preferably a mammal, for example, cattle, pigs, horses, goats, dogs, cats, rats, mice, rabbits, guinea pigs, human, and the like.

As used herein, the term "therapeutically effective amount" refers to an amount of an active ingredient or a pharmaceutical composition that induces a biological or medical response in a tissue system, animal or human, which is considered by a researcher, veterinarian, physician or other clinician. This includes an amount that induces alleviation of the symptoms of the disease or disorder to be treated. It is apparent to one of ordinary skill in the art that the therapeutically effective amount and the frequency of administration for the active ingredient of the present invention will vary depending on the desired effect. Therefore, the optimal dosage to be administered may be easily determined by one of ordinary skill in the art, and the range varies depending on the type of the disease, the severity of the disease, the content of an active ingredient and other ingredients contained in the composition, the type of formulation, the body weight, age, sex, and health condition of the patient, diet, administration time, administration method, excretion rate, and the like.

The present invention also provides a drug screening method using the microglia obtained by the method.

Important features of the microglia obtained by the present invention include the possibility of obtaining the production of a large quantity of cells, the maintenance of their properties even during cryopreservation, the possibility of maintaining the same cell population for a long period of time, and differentiation more similar to those of cells derived from a living body. This property is particularly suitable for simultaneous screening of multiple drugs, which requires a large quantity of cells in the same state and is the key to obtaining the same cells for a long period of time for repeated analysis thereof. It is very suitable for screening cells because a cell population with the same features in which key markers are maintained may be used continuously from the beginning to the end of the screening operation.

The drug is a drug for treating a disease selected from the group consisting of a neurodegenerative disease and an inflammatory degenerative disease, and exhibits a therapeutic effect on the neurodegenerative disease or inflammatory degenerative disease.

The neurodegenerative disease may include, for example, various nervous system diseases comprising Parkinson's disease, dementia, Alzheimer's disease, Huntington's disease, amyotrophic lateral sclerosis, memory loss, myasthenia gravis, progressive supranuclear palsy, multiple system atrophy, essential tremor, corticobasal degeneration, diffuse Lewy body disease and Pick disease, but is not limited thereto.

The inflammatory degenerative disease may include any one selected from the group consisting of dementia, Lewy body dementia, frontotemporal dementia, white matter degeneration, adrenoleukodystrophy, multiple sclerosis and Lou Gehrig's disease, but is not limited thereto.

All technical terms used in the present invention, unless otherwise defined, have the same meaning as commonly understood by one of ordinary skill in the art to which the present invention belongs. In addition, although preferred methods and samples are described herein, ones similar or equivalent thereto are also included in the scope of the present invention. The contents of all publications described herein as a reference are incorporated herein by reference.

### Mode for Carrying out the Invention

Hereinafter, the present application will be described in detail through examples. The following examples are only for illustrating the present application, and the scope of the present application is not limited to the following examples.

### Example 1: Differentiation from human induced pluripotent stem cells into microglia using yolk sac organoids

### [Experimental method]

### Culturing of human embryonic stem cells or human induced pluripotent stem cells

hESCs and hiPSCs were cultured based on the hESC research guidelines approved by the institutional review board (IRB) of Hanyang University (Seoul, Republic of Korea). The hESCs and hiPSCs used in this experiment are shown in Table 1 below.

**[Table 1]**

| **hiPSC line** | | | | | | | | |
|---|---|---|---|---|---|---|---|---|
| **Name used in this paper** | | **Original name** | | | **Karyotype** | | **Establishing institute** | |
| H9 | | WA09 | | | Female (46, XX) | | Wi cell | |

| **hiPSC line** | | | | | | | | |
|---|---|---|---|---|---|---|---|---|
| **Name used in this paper** | **Original name** | | **Source (Human)** | **Reprogramming factors** | | **Methods** | | **Establishing institute** |
| Epi-ips | episomal-ips1 | | Skin | OCT4. SOX2. KLF4, shp53 | | Episomal vector | | Hanyang Univ. (Our own) |

### Microglia differentiation using 3D organoids

hESCs and hiPSCs, which were normally cultured in an undifferentiated state, were detached using Accutase^{™} during passage culture, and undifferentiated human embryonic/dedifferentiated stem cells were seeded into a 96 well plate with a round bottom that does not attatch to the bottom at 20,000 cells/well to form organoids once.

In the medium composition of the first day, the generation of organoids was started with the additive conditions (BMP4 (Peprotech, #120-05ET 40 ng/ml) + activin A (Peprotech, #120-14P, 10 µg/ml) + ascorbic acid (Sigma, #A4544, 200 µM) + Y27632 (Sigma, #Y0503, 20 µM)) corresponding to Day 0 of culture (differentiation) of Table 2 in the NGD (neuroglia differentiation) medium of Table 3 below. The next day, development was induced by using a medium in which XAV939 (Sigma, #X3004, 2 µM) was additionally added to the components except for Y27632 (20 uM) among the components added on the first day to the same NGD medium (see Table 3). The culture medium was NGD until day 9, and development was induced by adding or subtracting the additives according to the concentration with reference to Table 3 for the addition components. The medium was replaced every day when the components were changed, and the medium was replaced every other day when the components were not changed. Correct formation of organoids was confirmed by the formation of a cell mass on days 4 to 8, and was checked by ejection into the outside of the cell after 12 days. On day 6 of organoid differentiation (culture), eight organoids formed one by one in a 96 well plate were grouped into each group and seeded and cultured into wells of a 6 well low binding plate. Development was induced under the N2 base media (Table 3) containing the components of Table 2 while rotating with an orbitary shaker (80 to 100 rpm). On day 20, the media containing the organoids was collected and centrifuged at 1000 g for 3 minutes to recover the ejected cells, and about 8 organoids per well of 6 wells were plated on a 6 well plate coated with PLO (poly-L ornithine; 15 µg/cm²)/Laminin (200 ng/cm²). Thereafter, the microglia attached to the 6 well plate were cultured according to the medium composition in Table 2, and the medium was replaced every 2-3 days.

**[Table 2]**

| | |
|---|---|
| Day 0 | Neuroglial Differentiation Media (recipe below) + BMP4 (Peprotech, #120-05ET 40 ng/ml) + Activin A (Peprotech, #120-14P, 10 µg/ml) + Ascorbic Acid (Sigma, #A4544, 200 µM) + Y27632 (Sigma, #Y0503, 20 µM) |
| Day 1~5 | Neuroglial Differentiation Media (recipe below) + BMP4 (Peprotech, #120-05ET 40 |
| | ng/ml) + Activin A (Peprotech, #120-14P, 10 µg/ml) + Ascorbic Acid (Sigma, #A4544, 200 µM) + XAV939 (Sigma, #X3004, 2 µM) |
| Day 6~9 | Neuroglial Differentiation Media (recipe below) + rhVEGF (Peprotech, #100-20 33 ng/ml) + bFGF (R&D systems, #233-FB, 20 ng/ml) + Ascorbic Acid (Sigma, #A4544, 200 µM) |
| Day 10~11 | N2 supplement base media (recipe below) + IL-34 (Peprotech, #200-34, 100 ng/ml) + M-CSF (Peprotech, #300-25, 25 ng/ml) + Ascorbic Acid (Sigma, #A4544, 200 µM) |
| Day 12~ End of culture | N2 supplement base media (recipe below) + IL-34 (Peprotech, #200-34, 100 ng/ml) + M-CSF (Peprotech, #300-25, 25 ng/ml) + TGF-B1 (Peprotech, #100-21, 50 ng/ml) + cholesterol (Sigma, #C8667, 1.5 µg/ml) + Ascorbic Acid (Sigma, #A4544, 200 µM) |

**[Table 3]**

| Neuroglial Differentiation Media (NGD) | N2 supplement Base Media |
|---|---|
| Neurobasal (100%) (Neurobasal, Gibco, #21103049) | N2 (100%) (N2 recipe as follows (1 L)) |
| GlutaMAX (Gibco, #35050061, 1:100) | D.W. 1 L |
| | DMEM-F12 12 g |
| Gem21 (Gemini Bio-products, #400-160, 1:100) | Sodium Bicarbonate 1.69 g |
| | D-glucose 1.55 g |
| Neuroplex N2 (Gemini Bio-products, #400-163, 1:100) | L-Glutamine 0.073 g |
| | Apo-transferrin 100 mg |
| AlbumaxI (Thermo Scientific, #11020-021, 0.2%) | Putrescine 0.1 mM |
| | Selenite 0.000030057803 mM |
| Sodium Chloride 50 mM | Progesterone 0.000020033708 |
| Sodium pyruvate 1 mM | mM |
| biotin (Sigma, #B4639, 3.5 ng/ml) | B27 (Gibco, #17504044, 1:50) |
| lactic syrup (Sigma, #L7900, 0.017%) | Insulin (Gibco, #12585014, 0.31 µl/ml) |
| | MEM-NEAA (Sigma, #M7145, 1:100) |
| | GlutaMAX (Gibco, #35050061, 1:100) |

### Method of chopping organoids

The method of chopping organoids and culturing them into single cells is as follows.

Organoids were finely divided (about 200 µm in diameter) with a thin syringe needle (30 gauge needle), was cultured with acutase for 10 minutes, and then attached to a PLO/Laminin-coated dish and cultured.

### Confirmation of mass proliferation and yield of yolk sac 3D organoid derived microglia

Microglia ejected from the yolk sac 3D organoids were plated into a 6 well plate every 2 days. The culture solution containing the yolk sac 3D organoids was collected, placed in a 15 ml conical tube, and centrifuged at 300 g for 2 minutes, and then the cell mass formed on the bottom of the tube was dissociated into single cells and then attached to a PLO/Laminin-coated 6 well plate. Thereafter, in addition to the cells ejected from the organoids, the culture solution containing new microglia proliferated from the cells in adherent culture in a 6 well plate was also collected and centrifuged every 2 days to separate the microglia, and then plated on a PLO/Laminin-coated 6 well plate again. From day 30, the number of microglia to be plated started to increase rapidly. As of 30 days, it is possible to obtain microglia about 6 times the number of cells in the first culture. For each plating, the number of cells was counted with a hemocytometer to calculate the yield. The yield was calculated based on the first 2×10⁶ cells.

### Immunocytochemistry analysis

Immunocytochemistry analysis is an analysis method that visualizes the target protein by attaching a primary antibody to the target protein and then attaching a secondary antibody to which fluorescent dye is attached to that antibody. The number of proteins that can be visualized is determined by the type of fluorescent dye of the secondary antibody. In this study, two or three types of fluorescent dye were used.

Sample was fixed with a fixative solution (4% paraformaldehyde/PBS) for 20 minutes, and then rinsed three times with PBS for 5 minutes each time. Blocking was performed for 40 minutes using a blocking buffer (1% BSA/PBS, 0.1% Triton X100), and then the primary antibody was dissolved in the same buffer and attached to the sample for 24 hours. The sample was rinsed three times with 0.1% BSA/PBS, and then the secondary antibody was dissolved in the same buffer and attached for 1 hour. Thereafter, the sample was rinsed three times with 0.1% BSA/PBS, and then rinsed once with D.W., and mounted on a cover slide with a mounting solution (Vectashield, Vector Lab).

The organoid section immunocytochemistry analysis was performed as follows. The organoid was fixed in a fixative solution for more than 30 minutes, and then rinsed three times with PBS for 10 minutes each time, and then cultured in a 30% sucrose solution for 24 hours, and dehydrated. The dehydrated sample was frozen in an OCT (optimal cutting temperature) compound and then cut to a thickness of 10 to 12 µm with a microtome, and the above blocking process was performed.

### RT-PCR

RNA of the sample to be analyzed was extracted using Trizol, and after RNA extraction was completed, cDNA was synthesized, and RT-PCR was performed. This is an experimental method that can compare the relative amount of RNA present between samples, and in this study, it was used to confirm the expression of a marker protein in a specific sample through comparison between several samples.

The RNA extraction method is as follows. The cells were lysed in 1 ml of Trizol for 5 minutes, and 200 µl of chloroform was added, mixed with shaking, and then let stand for 2-3 minutes. Centrifugation was performed at 12000 Xg at 4°C for 15 minutes. After centrifugation, the transparent part of the supernatant was collected (400 to 500 µl) and transferred to a new tube, and 500 µl of isopropanol was added thereto and then mixed. After incubation for 10 minutes, centrifugation was performed at 12000 Xg at 4°C for 10 minutes.

The supernatant was removed except for a small amount of RNA mass that remained at the bottom of the tube, and then 1 ml of 75% ethanol solution was added and mixed with the mass. Centrifugation was performed at 7500 Xg at 4°C for 5 minutes. The supernatant was removed, and then only the RNA mass was left, and then the lid was opened and air-dried. The dried RNA was dissolved in RNase-free water, and then the concentration thereof was measured.

The cDNA synthesis method is as follows. 2 µg of RNA was used for cDNA synthesis. First-strand cDNA synthesis was performed using a random primer (Invitrogen), and reaction was performed by a PCR machine at 75°C for 15 minutes. Thereafter, incubation was performed on ice for 2 minutes, and then first-strand buffer (Invitrogen), DTT (Invitrogen), and RNasin (Promega) were added, and reaction was performed at 25°C for 15 minutes, at 42°C for 50 minutes, and at 70°C for 15 minutes.

The prepared cDNA was a total of 20 µl, which was diluted 10-fold and used for RT-PCR. SYBR green master mix (Bio-rad) was used for the reaction, and 2 µl of cDNA was reacted each time.

### Confirmation of progenitor cell generation

The microglia mass, which constantly produces microglia, was stained with IBA1, and if positive, it was identified as a microglia progenitor cell. The proliferating cells were also identified through microscopic monitoring in real time. In addition, it was confirmed through the expression of the PU.1 marker on day 14 of differentiation of an organoid that ejects microglia.

### Confirmation of purity and yield

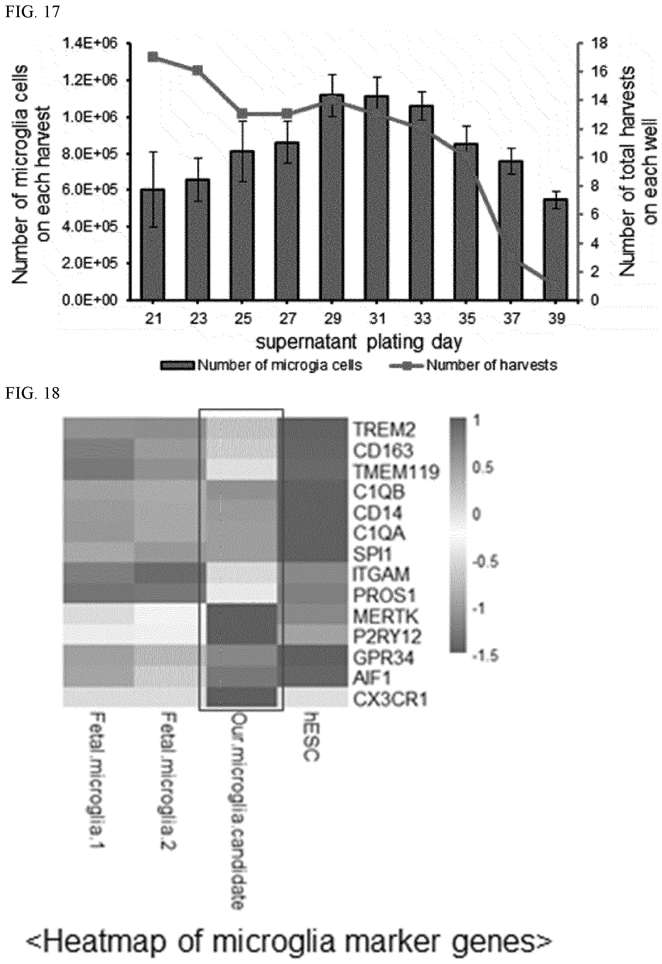

Purity was confirmed by staining of marker proteins (Iba1, CX3CR1, etc.) of microglia by immunocytochemistry analysis. The generation and ejection of cells expressing the marker protein were counted by date, and the yield was calculated compared to the cells plated initially (FIG. 17).

The cells (microglia + microglia progenitor cells) released from the yolk sac-mimic organoids were collected once every 2 days and attached to one well (9.6 cm²) of a 6 well plate from day 21 to day 39, and then the microglia floating in each well were harvested again once every 2 days and measured.

### Confirmation of cell function (phagocytic activity)

The first cellular phagocytic activity was confirmed by treating the cells with the fluorescence-labeled microspheres and confirming the microspheres that entered the cells. 1x10^4 cells per well were attached to a 24 well plate and treated with 3x10^5 beads one day later. The next day, the cells were washed three times with PBS to remove the microspheres that were attached to the surface and did not enter the cells, and then the cells were fixed, and immunofluorescence staining was performed.

The second cellular phagocytic activity was confirmed by attaching PFF (Pre-formed Fibril) to pHrodo and treating the microglia. pHrodo is a substance that emits red fluorescence in an acidic environment. When pHrodo is phagocytosed by cells, it shows red fluorescence. PFF is a substance to be phagocytosed by a phagocyte, such as microglia. pHrodo (Invitrogen, #P36600) was attached to PFF and the microglia were treated therewith, and then the amount of fluorescence was measured for each hour to confirm the phagocytic activity of the microglia. In an experiment method, 2.86 µl of 5 mg/ml PFF was mixed with 1 µl of pHrodo in a 1.5 ml tube and then stored at room temperature for 1 hour in a state of blocking light. 1 ml of PBS was added, and centrifugation was performed at 14000 rpm for 1 minute, and then the supernatant was removed while leaving only a dark purple mass at the bottom of the tube. 1 ml of PBS was added, and the mass was dissolved, and centrifugation was performed again at 14000 rpm for 1 minute. This was repeated once more. The purple mass was released with 1 ml of the culture solution, and then 250 µl of the cells being grown in a 24 well plate was aliquoted and added into each well. Observation was started 1.5 hours after treatment.

### Cryopreservation of 3D organoid derived microglia

At 30 days of culture, 5×10⁵ cells of the organoid derived microglia were frozen at - 196°C with a freezing solution composed of 90% culture solution and 10% DMSO and then thawed after 100 days to confirm the survival rate.

### [Experiment result]

### Preparation of yolk sac organoids from human embryonic stem cells

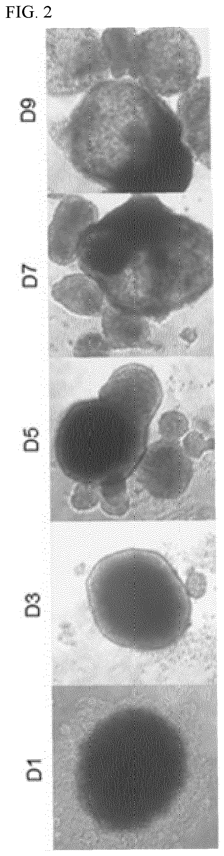

In FIG. 2, it was confirmed that the yolk sac organoids were prepared by an optical microscope. FIG. 2 is a photograph of the organoids from day 1 to day 9 after differentiation (culture) derived from iPSCs that are developing in the yolk sac. It was confirmed that the semi-transparent cyst structure resembles the actual yolk sac structure.

### Confirmation of expression of primitive streak HSC marker

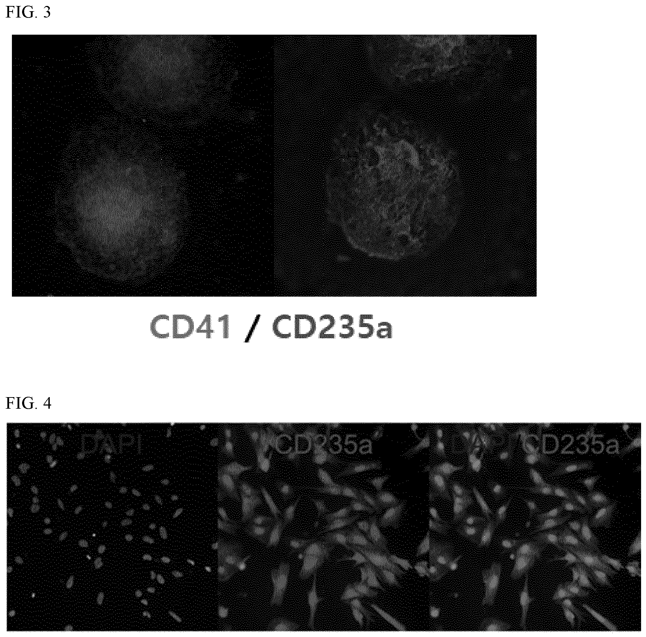

In FIG. 3, in order to confirm whether primitive hematopoietic stem cells (primitive HSCs) are actually generated inside the yolk sac organoids, the organoids were frozen and then cut into sections, and the cross-section was analyzed. On day 14 of differentiation, the yolk sac organoids were fixed with a fixative solution and confirmed by immunocytochemistry analysis. It was confirmed whether the primitive streak HSC markers CD41 and CD235a were expressed inside. Since microglia originate from primitive streak HSCs among primitive streak HSCs and definitive streak HSCs, the existence of a CD235a protein, which is expressed in primitive streak HSCs but not expressed in definitive streak HSCs, was confirmed by immunocytochemistry analysis.

In addition, in FIG. 4, from the cross-section of the organoids, as well as from analysis after adherent culture by chopping the organoids to a single cell level, it was confirmed by immunocytochemistry analysis that more than 90% of the cells expressed CD235a. On day 14 of differentiation, the yolk sac organoids were chopped to a single cell level using accutase and then two-dimensionally plated, and the properties of the cells inside the organoids were analyzed.

### Expression of microglia marker

On day 14 of differentiation (culture), RNA of the yolk sac organoids was collected, and the amount of gene markers expressed in the early stage of microglia differentiation was confirmed by RT-PCR (CD235a; primitive HSC, CD34; HSC, PU.1; myeloid-associated transcription factor, TREM2; microglia-enriched protein).

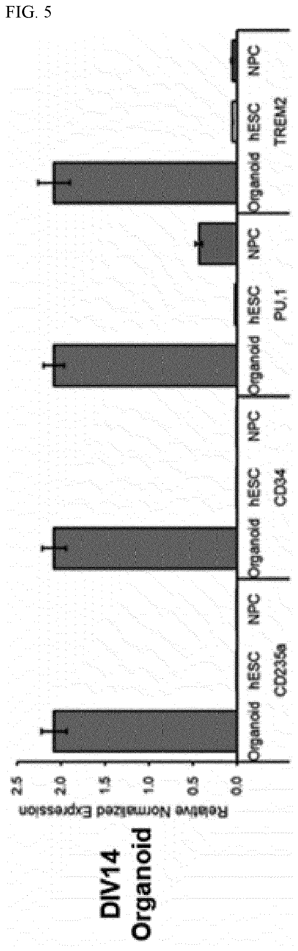

As shown in FIG. 5, it was observed that a significantly higher amount of gene markers was expressed in the yolk sac organoids compared to the control group, embryonic stem cells (H9 hESCs) and other cell types (neural progenitor cells, NPCs). This indicates that the cells in the early stage of microglia differentiation are highly concentrated in the organoids.

### Confirmation of change in shape of cells ejected from organoids

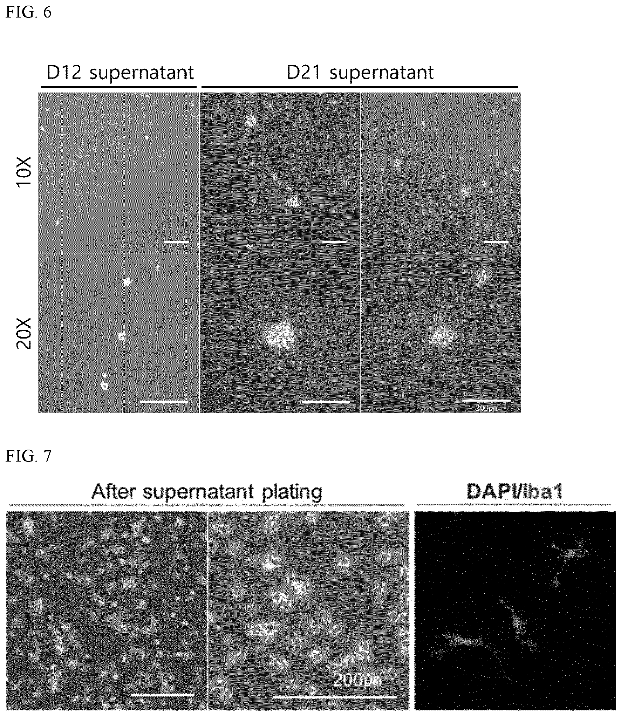

On day 10 of differentiation (culture), the medium was replaced with the N2 medium containing M-CSF and IL-34, which mimics the environment in which microglia escape from the yolk sac and penetrate into the brain. When the medium was replaced, the cells began to be ejected from the organoids. It was confirmed that two days after replacing the medium (12 days of differentiation), the shape of the ejected cells was mostly circular, whereas 11 days after replacing the medium (21 days of differentiation), the shape of the ejected cells was similar to that of microglia, which had the shape of the extended branch [FIG. 6]. Therefore, when the ejected cells were collected and placed on a PLO/laminin-coated cell culture dish, the shape shown in FIG. 7 is obtained.

FIG. 7 is a photograph taken after collecting the microglia ejected from the organoids that were differentiated (cultured) for 24 days and attaching to the cell culture dish. The bright field photograph on the left was taken the next day after cell attachment, and the shape was very similar to that of microglia. After 6 days, the expression of Iba1, a microglia marker, was confirmed by fluorescence staining. As a result, the proportion of IbaI-expressing cells in the total cells was about 90%. The microglia could be differentiated with a high purity without sorting

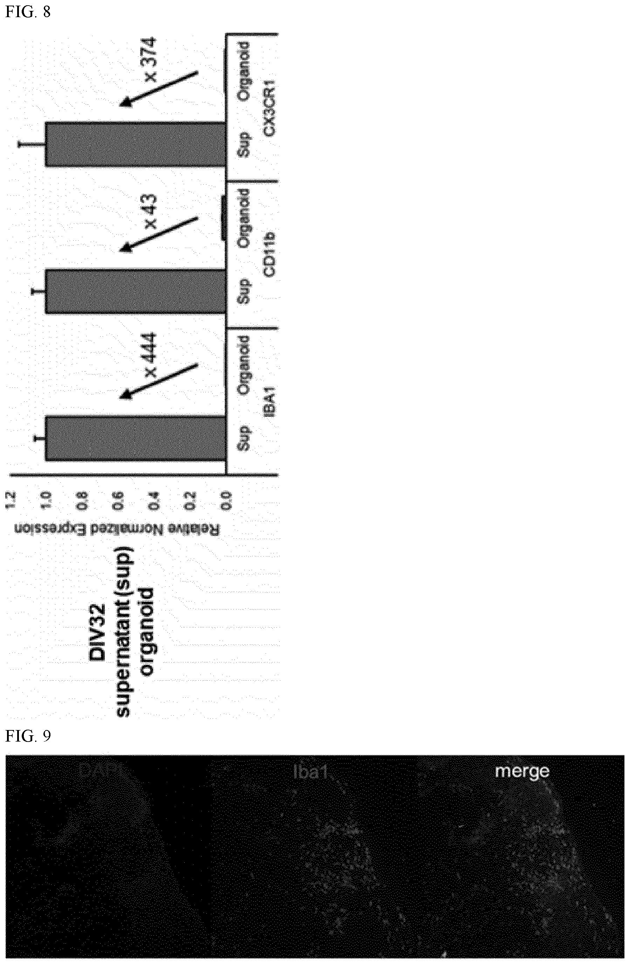

The expression of the mature microglia marker gene (Iba1; microglia, CD11b; macrophage and microglia, CX3CR1; microglia-enriched protein) in each of the microglia ejected on day 32 of differentiation (culture) (supernatant, sup) and the yolk sac organoids was confirmed by qPCR. As a result, it was confirmed that the mature microglia marker gene was as high as at least 43 times and at most 444 times higher in the cells ejected into the supernatant than in the yolk sac sphere [FIG. 8].

Therefore, the mature microglia can escape out, and the shape is very similar to that of microglia, and the ejected microglia express a significant amount of mature microglia gene markers. Thus, it has been shown that microglia can be successfully obtained by a method of preparing the yolk sac organoids and obtaining the ejected cells.

FIG. 9 illustrates a result obtained by analyzing the cross-section of the yolk sac organoids that were differentiated (cultured) for day 38 by immunocytochemistry analysis. It was confirmed that a cyst structure in which cells (DAPI) are not present inside the organoid is present (actually a structure similar to the yolk sac), and microglia (Iba1) are distributed around it. In particular, Iba1 was mostly distributed on the surface of the organoid rather than inside the cyst. This once again showed that it similarly mimics the situation of real tissues in which microglia escape from the yolk sac.

### Generation of microglia progenitor cells

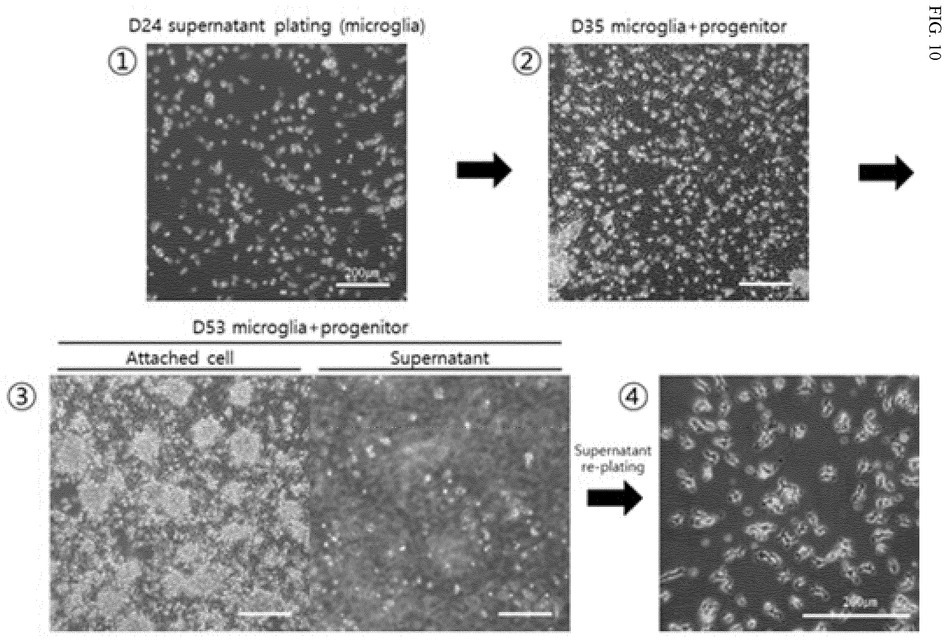

In addition to the cells ejected from the organoids, the culture solution containing new microglia proliferated from the cells in adherent culture in a 6 well plate was also collected and centrifuged every 2 days to separate the microglia, and then plated on a PLO/Laminin-coated 6 well plate again. From day 30 of differentiation (culture), the number of microglia to be plated started to increase rapidly. In FIG. 10, ①: a photograph of cells ejected from the organoids plated on a cell culture dish on day 24 of differentiation (culture); ②: a photograph when further cultured for +11 days in the same well as in photograph ①; ③: a photograph when further cultured for +29 days in the same well as in photograph ① (cells attached to the dish and floating cells); ④: a photograph of the cells by collecting the floating cells again in photograph ③ and plating on a cell culture dish.

When cells were collected from the yolk sac organoids on day 24 of differentiation (culture), microglia purity of more than 90% was observed. In the rest, a cell population presumed to be microglia progenitor cells was observed in addition to cells having a typical microglia shape (Photograph ① in FIG. 10). These cells have a more excellent proliferative capacity than the microglia (Photograph (2) in FIG. 10). After about day 30, a cluster of cells was formed thereon, and the shape of the cells forming the cluster was very similar to that of the microglia. In addition, cells presumed to be microglia were floated in the culture solution. When these cells were collected and plated again, it was confirmed that they were microglia (Photograph ④ in FIG. 10).

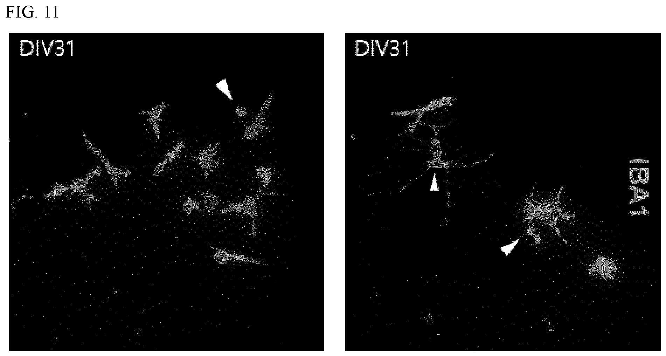

The microglia candidate cell population having an excellent proliferative capacity does not proliferate immediately after escaping from the organoids, but proliferates after a certain period of time (about 30 days of differentiation). IBA1 staining confirmed the possibility that proliferating cells were microglia progenitor cells on day 31 of differentiation (white arrow in FIG. 11 indicates proliferating cells). The proliferating cells were identified by monitoring the proliferation situation of the cells in real time under a microscope.

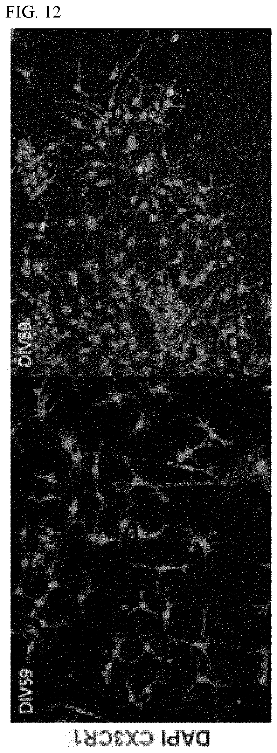

It was confirmed by immunocytochemistry analysis that both cells having an excellent proliferative capacity, and microglia expressed CX3CR1, an important marker of microglia (left in FIG. 12; microglia, right in FIG. 12; proliferating cells). CX3CR1 is a representative marker of homeostatic microglia. Based on these results, this proliferating cell population was presumed to be microglia progenitor cells.

### Confirmation of function of prepared cells as microglia

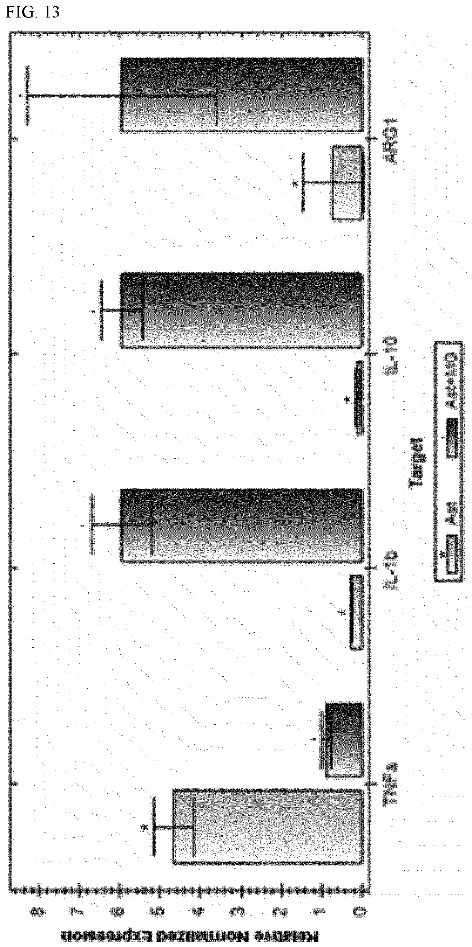

FIG. 13 illustrates a result obtained by confirming the response to toxic stimuli (LPS) by inflammatory response markers when astrocytes are cultured alone or when astrocytes and microglia are co-cultured. It is known that astrocytes have weaker expression of TLR2,4 that recognizes LPS stimulation compared to microglia. Therefore, the experiment was designed as above because it was expected that there would be a difference in the sensitivity of microglia to toxic stimuli during co-culture. When co-cultured with microglia, it was observed that the expression of TNFa, an important marker of the inflammatory response, was decreased, and the expression of IL-10 and Arg1, the major markers of the anti-inflammatory response, was increased. Astrocytes were cultured alone as a control group. When astrocytes were co-cultured with microglia, it was observed that toxicity sensitivity of astrocytes was induced by stimulation of a change in the state of microglia under LPS stimulation. Therefore, it was confirmed that the microglia function normally.

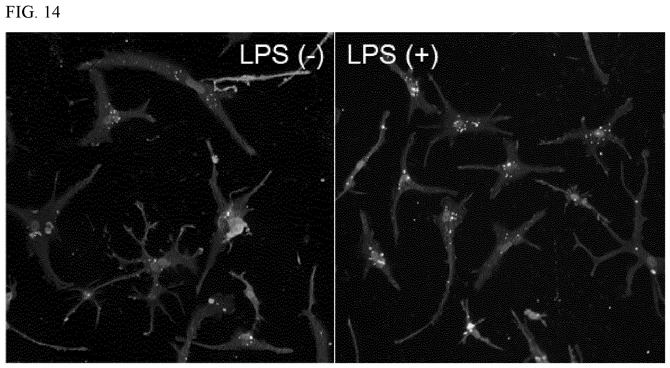

The phagocytic activity of the microglia was confirmed using fluorescent beads. It was observed that they phagocytosed a lot of fluorescent beads. Therefore, it was confirmed that they well induce phagocytosis, a major function of microglia [FIG. 14].

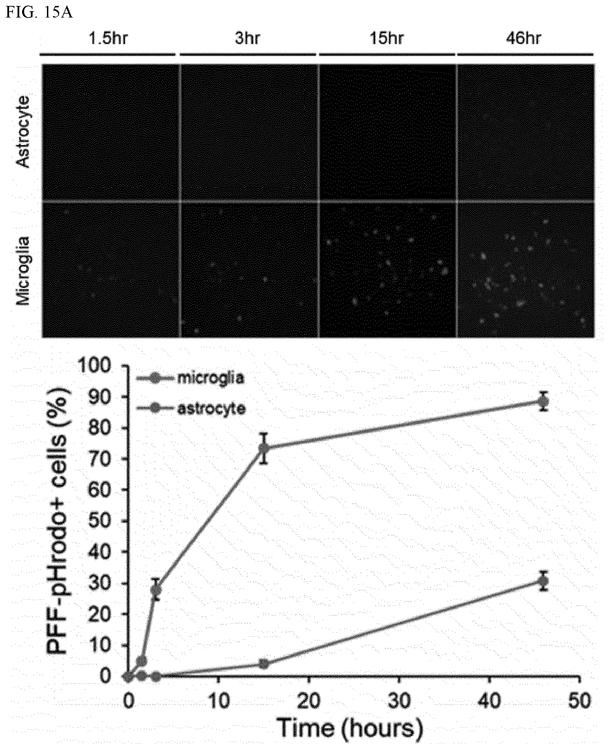

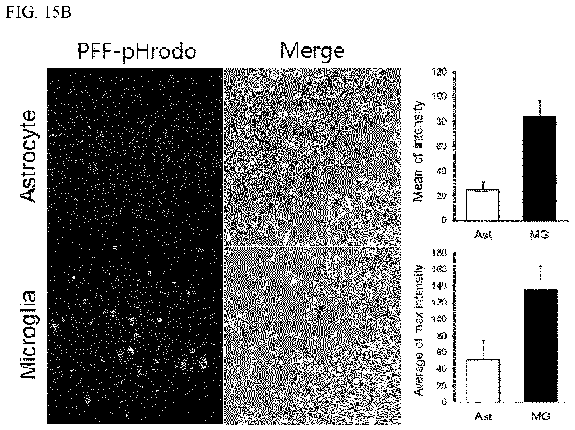

The phagocytic activity of the microglia was confirmed using pHrodo^{™} Green and Red Amine-Reactive Labels. As a result of treating the cells with pHrodo (a substance that is tagged to PFF protein, which becomes red when it enters the cell due to phagocytosis), it was observed that microglia expressed more red color in a faster time compared to astrocytes (FIG. 15A). As a result of quantifying the amount of pHrodo accumulated in the cells through intensity analysis, it was confirmed that the amount was three times greater in microglia (FIG. 15B).

### Confirmation of possibility of microglia cryopreservation

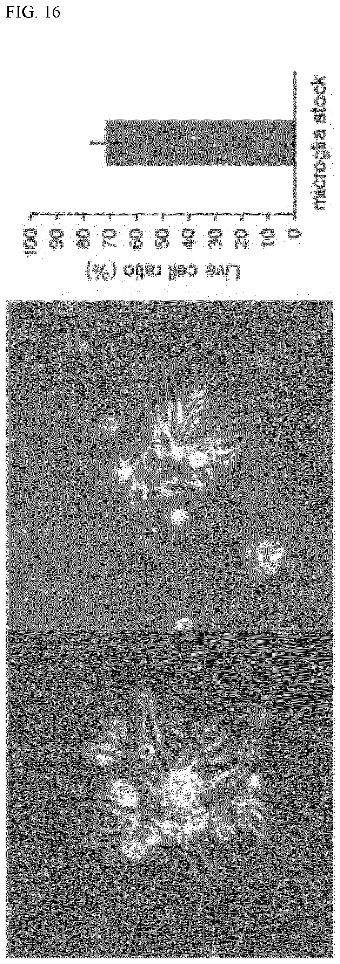

Microglia were cryopreserved at -196°C for 100 days and then thawed again and grown. As a result, it was confirmed that 71.5% of the cells were alive [FIG. 16].

### Confirmation of obtained microglia yield

The cells (microglia + microglia progenitor cells) released from the organoids were collected once every 2 days and attached to one well (9.6 cm²) of a 6 well plate from day 21 to day 39, and then the microglia floating in each well were harvested again once every 2 days and measured. The results are shown in FIG. 17. (Since microglia progenitor cells are attached to wells, the number of only floating cells was measured. Microglia are continuously generated by microglia progenitor cells and can be harvested several times in one well.) The number of microglia that could be obtained at one time was the largest in the wells that were attached around day 30, and the earlier the cells were attached in the wells, the more cells could be obtained.

5.5 to 6.5 times the number of mature microglia can be obtained on day 30 compared to the initial number of cells (starting with 2×10⁶ iPS cells), and when accumulated by day 40, the total amount of the harvested microglia can be obtained in an amount of about 50 times. This is more than 4 times (Non-Patent Document 1) and 30 to 40 times (Non-Patent Document 2) compared to the prior art.

### Comparative analysis of gene expression with actual human fetal microglia

FIG. 18 illustrates a result obtained by comparative analysis of the gene expression data (public data) of human fetal microglia through Non-Patent Document 1 and the gene expression data of microglia (red box) and H9 embryonic stem cells (hESC) obtained according to the present invention. All the genes shown in the heatmap are genes used as markers for microglia. It was confirmed that the invented microglia showed a gene expression pattern very similar to that of fetal microglia.

## Claims

1. A medium composition for inducing differentiation of microglia, the medium composition further comprising a compound represented by Formula 1 below:

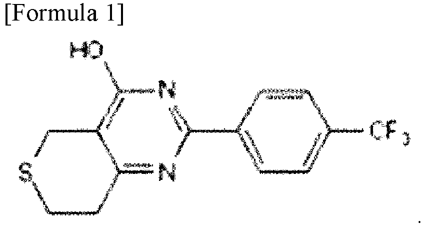

2. The composition according to claim 1, wherein the medium for inducing differentiation of microglia is an NGD (neuroglia differentiation) medium.

3. The composition according to claim 1, wherein the medium for inducing differentiation of microglia comprises BMP4 (bone morphogenetic protein 4) and activin A.

4. The composition according to claim 3, wherein bFGF (basic fibroblast growth factor) and VEGF (vascular endothelial growth factor) are further comprised.

5. A method for differentiation of microglia to obtain a large quantity of microglia, the method comprising:
preparing yolk sac-mimic organoids from human pluripotent stem cells; and
replacing an NGD (neuroglia differentiation) medium with an N2 supplement medium while culturing the organoids in the NGD medium;
wherein a compound represented by Formula 1 below is further included in the NGD medium when culturing in the NGD medium:

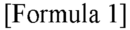

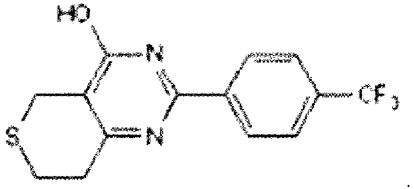

6. The differentiation method according to claim 5, wherein the NGD medium is replaced with an N2 supplement medium from day 9 to day 11 of culture.

7. The differentiation method according to claim 5, wherein BMP4 (bone morphogenetic protein 4) and activin A are comprised in the NGD medium.

8. The differentiation method according to claim 5, wherein bFGF (basic fibroblast growth factor) and VEGF (vascular endothelial growth factor) are further comprised in the NGD medium.

9. The differentiation method according to claim 5, wherein M-CSF (macrophage colony stimulating factor) and IL-34 (interleukin 34) are further comprised in the N2 supplement medium.

10. The differentiation method according to claim 5, wherein cholesterol and TGF-β1 (transforming growth factor beta 1) are further comprised in the N2 medium.

11. The differentiation method according to claim 5, wherein the differentiation method further comprises performing cryopreservation.

12. The differentiation method according to claim 5, wherein the culture is performed in an incubator of 1-10% CO₂.

13. The differentiation method according to claim 5, wherein a cell sorting process is not performed separately.

14. A composition for preventing or treating a neurodegenerative disease or an inflammatory degenerative disease, the composition comprising an anti-inflammatory cytokine derived from microglia differentiated by the method according to claim 5.

15. The composition according to claim 14, wherein the anti-inflammatory cytokine is at least one selected from the group consisting of IL-4, IL-10, IL-33, TNF-β and IGF-1.

16. The composition according to claim 14, wherein the neurodegenerative disease is any one selected from the group consisting of Parkinson's disease, dementia, Alzheimer's disease, Huntington's disease, amyotrophic lateral sclerosis, memory loss, myasthenia gravis, progressive supranuclear palsy, multiple system atrophy, essential tremor, corticobasal degeneration, diffuse Lewy body disease and Pick disease.

17. The composition according to claim 14, wherein the inflammatory degenerative disease is any one selected from the group consisting of dementia, Lewy body dementia, frontotemporal dementia, white matter degeneration, adrenoleukodystrophy, multiple sclerosis and Lou Gehrig's disease.

18. A drug screening method using microglia differentiated by the method according to claim 5.

19. The drug screening method according to claim 18, wherein the drug treats a neurodegenerative disease or an inflammatory degenerative disease.

20. The drug screening method according to claim 19, wherein the neurodegenerative disease is any one selected from the group consisting of Parkinson's disease, dementia, Alzheimer's disease, Huntington's disease, amyotrophic lateral sclerosis, memory loss, myasthenia gravis, progressive supranuclear palsy, multiple system atrophy, essential tremor, corticobasal degeneration, diffuse Lewy body disease and Pick disease.

21. The drug screening method according to claim 19, wherein the inflammatory degenerative disease is any one selected from the group consisting of dementia, Lewy body dementia, frontotemporal dementia, white matter degeneration, adrenoleukodystrophy, multiple sclerosis and Lou Gehrig's disease.

22. A method for treating a neurodegenerative disease or an inflammatory degenerative disease, comprising administering to a subject a therapeutically effective amount of a microglia-derived anti-inflammatory cytokine.

23. The treatment method according to claim 22, wherein the anti-inflammatory cytokine is at least one selected from the group consisting of IL-4, IL-10, IL-33, TNF-β and IGF-1.

24. The treatment method according to claim 22, wherein the neurodegenerative disease is any one selected from the group consisting of Parkinson's disease, dementia, Alzheimer's disease, Huntington's disease, amyotrophic lateral sclerosis, memory loss, myasthenia gravis, progressive supranuclear palsy, multiple system atrophy, essential tremor, corticobasal degeneration, diffuse Lewy body disease and Pick disease.

25. The treatment method according to claim 22, wherein the inflammatory degenerative disease is any one selected from the group consisting of dementia, Lewy body dementia, frontotemporal dementia, white matter degeneration, adrenoleukodystrophy, multiple sclerosis and Lou Gehrig's disease.
